# EUROPEAN PATENT APPLICATION

(11) **EP 4 520 361 A1**
(43) Date of publication of application: **12.03.2025**
(21) Application number: 23796496.0
(22) Date of filing: 27.04.2023
(51) Int. Cl.: A61M 1/22, A61M 1/34

(54) **MEDICAL INSTRUMENT AND MANUFACTURING METHOD THEREOF**

(30) Priority: 30.04.2022 JP 2022075623
(71) Applicant: Keio University, Tokyo, 108-8345 (JP)
(72) Inventor: MIKI Norihisa, Yokohama-shi, Kanagawa 223-8522 (JP); OTA Takashi, Yokohama-shi, Kanagawa 223-8522 (JP)
(74) Representative: Peters, Sebastian Martinus
(86) International application number: PCT/JP2023/016703
(87) International publication number: WO 2023/210761

(57) **Abstract**

A medical instrument with little risk of clot formation even when blood is flowed therethrough, said medical instrument (1) comprising a plurality of blood channel layers (3), wherein the plurality of blood channel layers (3) each have a plate-shaped main body (5), a groove (9) for forming a blood channel (7), and a resin layer (11) formed on the side wall of the groove (9). A manufacturing method of the medical instrument comprises: a resin applying step for applying a resin to the groove (9) of the plate-shaped main body (5) having the groove (9) for forming the blood channel (7); a bonding step for sandwiching the plate-shaped main body (5) between a first film (41) and a second film (43) and thus bonding the plate-shaped main body (5), the first film (41) and the second film (43); and a post-curing step for, after the bonding step, curing the resin applied to the groove (9) in the resin applying step.

## Description

### TECHNICAL FIELD

The present invention relates to a medical instrument and a manufacturing method thereof. In more detail, the present invention relates to a medical instrument including a blood flow channel with a wall covered with a resin and a manufacturing method thereof.

### BACKGROUND ART

Japanese Patent Publication No. 2020-134150 describes a micro flow channel chip. Japanese Patent Publication No. 2017-158814 describes a blood filtration device. Japanese Patent Publication No. 2020-22524 describes a plasma separation device. The cross-sections of these flow channels are rectangular. Thus, when blood is flowed through the flow channels, clots are likely to form. These flow channels are basically obtained by machining metal. Thus, these flow channels may have a nonuniformity when machining metal or may not have a high affinity for blood.

### CITATION LIST

### PATENT LITERATURE

Patent Literature 1: Japanese Patent Publication No. 2020-134150.
Patent Literature 2: Japanese Patent Publication No. 2017-158814.
Patent Literature 3: Japanese Patent Publication No. 2020-22524.

### SUMMARY OF INVENTION

### TECHNICAL PROBLEM

The objective of the present invention is to provide a medical instrument with little risk of clot formation even when blood is flowed therethrough, and a manufacturing method thereof.

### SOLUTION TO PROBLEM

The problems described above can basically be solved by coating a side wall of a groove formed on a substrate with a resin, thereby making corners rounded and reducing the obstruction to the flow of blood.

The first invention relates to a medical instrument. The medical instrument 1 includes a blood flow channel layer 3. The blood flow channel layer 3 is usually multilayered. The blood flow channel layer 3 is a layer including a flow channel through which blood is flowed. Each blood flow channel layer 3 includes a plate-shaped main body 5, a groove 9 and a resin layer 11. The resin layer 11 is an element formed by a resin on a side wall of the groove 9.

The next invention relates to a manufacturing method of a medical instrument. The method includes a resin applying step (S101), a bonding step (S103) and a post-curing step (S104).

### ADVANTAGEOUS EFFECTS OF INVENTION

The present invention can provide a medical instrument with little risk of clot formation even when blood is flowed therethrough, and a manufacturing method thereof.

### BRIEF DESCRIPTION OF DRAWINGS

FIG. 1 is a conceptual diagram illustrating an example of a medical instrument including a blood flow channel layer. FIG. 1 (a) is an example of the external appearance of a device. FIG. 1 (b) is an example of the blood flow channel layer. FIG. 1 (c) is an example of a blood flow channel.
FIG. 2 is a conceptual diagram for describing a resin layer.
FIG. 3 is a conceptual diagram for describing the layer structure of a blood filtration device and a blood separation device.

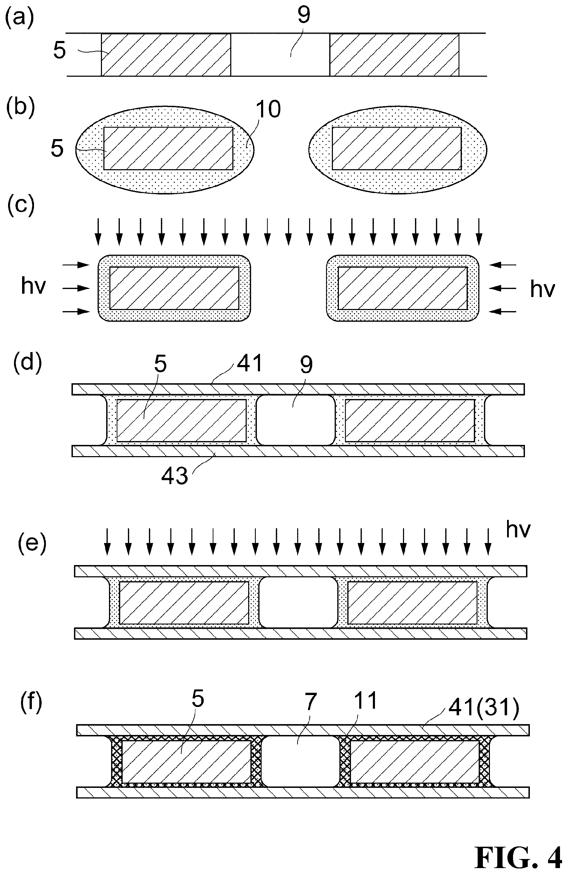

FIG. 4 is a conceptual diagram for describing manufacturing processes of the medical instrument. FIG. 4 (a) is a diagram illustrating an example of the cross-section of a plate-shaped main body including a groove. FIG. 4 (b) is a conceptual diagram illustrating the groove being applied with a resin. FIG. 4 (c) is a diagram illustrating an example of a pre-curing step. FIG. 4 (d) is a conceptual diagram illustrating the plate-shaped main body being sandwiched between a first film and a second film. FIG. 4 (e) is a conceptual diagram illustrating how the resin applied to the groove is cured by irradiating light onto the plate-shaped main body obtained in a bonding step that is sandwiched between the first film and the second film. FIG. 4 (f) is a conceptual diagram illustrating a partial view of the medical instrument obtained.
FIG. 5 is a photograph in lieu of a drawing of a groove section. FIG. 5 (a) is a photograph in lieu of a drawing of the groove section obtained in an example. FIG. 5 (b) is a photograph in lieu of a drawing of the groove section where the resin layer is not provided.
FIG. 6 is a photograph in lieu of a drawing showing the appearance of the medical instrument obtained in the example.
FIG. 7 is a photograph in lieu of a drawing showing how blood is filtered using the medical instrument. FIG. 7 (a) is a photograph in lieu of a drawing showing how the blood flow channel layer is removed after using the medical instrument obtained in the example. FIG. 7 (b) is a photograph in lieu of a drawing showing how the medical instrument that does not include the resin layer is used.

### DESCRIPTION OF EMBODIMENTS

An embodiment of the present invention will be explained below with reference to the drawings. The present invention is not limited to the embodiment described below and incorporates modifications from the embodiment below to the extent that they are obvious to those skilled in the art.

### Medical instrument 1

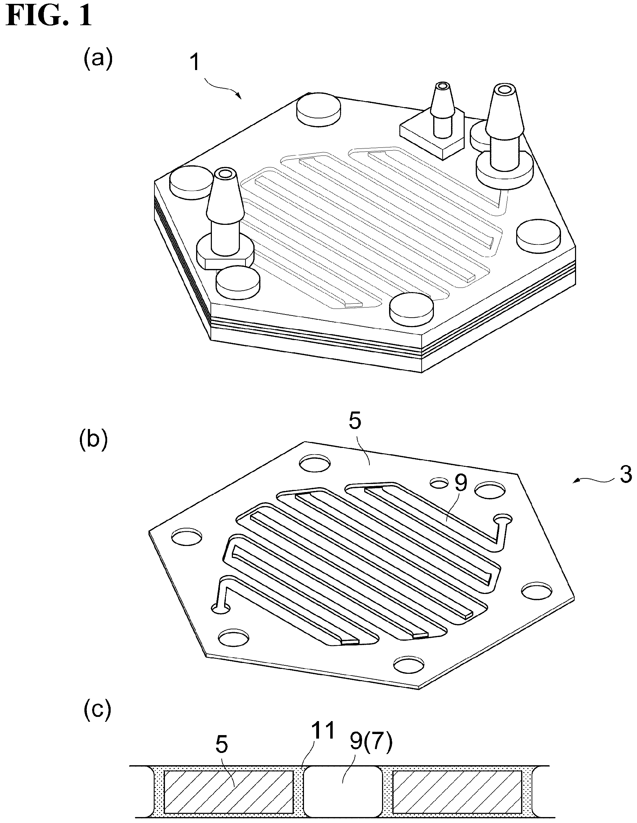

The first invention of the present description relates to the medical instrument 1. FIG. 1 is a conceptual diagram illustrating an example of a medical instrument including a blood flow channel layer. The present invention is not limited to the medical instrument illustrated in FIG. 1. FIG. 1 (a) is an example of the external appearance of a device. FIG. 1 (b) is an example of the blood channel layer. FIG. 1 (c) is an example of a blood flow channel. As illustrated in FIG. 1, the medical instrument 1 includes the blood flow channel layer 3. The blood flow channel layer 3 is usually multilayered. The blood flow channel layer 3 is a layer including a flow channel through which blood is flowed. Each blood flow channel layer 3 includes a plate-shaped main body 5, a groove 9 and a resin layer 11. Examples of the medical instrument 1 include an implantable artificial kidney, a portable artificial kidney, a hemodialysis machine, a blood filtration device and an extracorporeal membrane oxygenator. The blood filtration device also includes a continuous hemodiafiltration device. Components other than the resin layer 11 can be the ones described in Japanese Patent Publication Nos. 2017-158814 and 2020-22524, as appropriate.

For example, a blood filtration device 1 includes a main body including an external shape of a regular hexagon. The main body includes a stacked structure formed by stacking a plurality of plate-shaped members. A blood inlet, a blood outlet and a filtrate outlet are provided on one side of the main body in the stacking direction. The blood inlet allows blood to be flowed into the main body, and the blood outlet allows blood to be flowed out of the main body. The filtrate outlet discharges filtrate that is filtered in the main body to be extracted from blood. Each of the blood inlet, the blood outlet and the filtrate outlet 5 is in the form of a connector to which tubing can be connected.

The blood flow channel layer includes the blood flow channel 7 through which blood is flowed. A filtrate flow channel layer includes a filtrate flow channel through which filtrate is flowed. In the main body, the blood flow channel layer and the filtrate flow channel layer are arranged in a position where the blood flow channel and the filtrate flow channel face each other via a filtration film between them, and blood is filtered by the filtration film in the process of blood being flowed through the blood flow channel, and plasma including harmful substances in blood and excessive water is flowed into the filtrate flow channel and discharged as filtrate from the filtrate outlet.

### Blood flow channel layer 3

The blood flow channel layer 3 is generally plate-shaped and includes the groove 9 in the plate-shaped main body 5, which serves as the blood flow channel. The medical instrument usually includes a plurality of blood flow channel layers. For example, the number of stacked layers may be 1 layer or more and 120 layers or less, 2 layers or more and 60 layers or less, 5 layers or more and 30 layers or less, or 5 layers or more and 20 layers or less. Meanwhile, for example, for humans (especially adults) weighing 20 kg or more and 100 kg or less, the number of stacked layers may be 1 layer or more and 300 layers or less, 1 layer or more and 240 layers or less, 1 layer or more and 120 layers or less, or 2 layers or more and 60 layers or less.

### Plate-shaped main body 5

The plate-shaped main body 5 can be made of a metal material such as titanium or stainless steel, a synthetic resin material such as plastic, or a ceramic material such as silicon or glass.

The groove 9 is formed in the plate-shaped main body 5 and is an element for forming the blood flow channel 7.

The width of the flow channel may be 0.01 mm or more and 1 cm or less, 0.1 mm or more and 5 mm or less, or 0.5 mm or more and 4 mm or less. The thickness of the flow channel is adjusted as appropriate according to the thickness of the device and the number of stacked layers, although if the flow channel is too thick, the flow rate of blood becomes too high, and if the flow channel is too thin, the flow of blood becomes stagnant, resulting in a high risk of clogging of the flow channel due to the deposition of impurities. From this perspective, the thickness of the flow channel is preferably 0.01 mm or more and 1 cm or less, may be 0.05 mm or more and 5 mm or less, or may be 0.1 mm or more and 3 mm or less.

### Resin layer 11

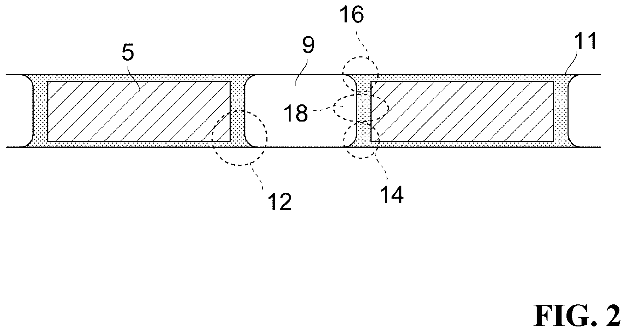

The resin layer 11 is an element formed by a resin on a side wall of the groove 9. The medical instrument includes the resin layer 11 formed on the side wall of the groove 9, thereby preventing/reducing the exposure of the groove and the contact of the groove with blood. FIG. 2 is a conceptual diagram for describing the resin layer. The resin layer 11 is preferably present at least in a corner area 12 of the groove to make the shape of the groove smooth. Therefore, the medical instrument of the present invention can reduce the surface roughness of a blood-contacting surface independent of the machining roughness of the groove. The resin layer 11 preferably covers the side wall of the groove. The resin layer 11 may cover the entire groove (both sides of the wall, and the top and bottom (left and right) sides of the wall). In other words, the entire wall composing the blood flow channel may be covered by the resin layer. Since the plate-shaped main body 5 does not contact with blood, the medical instrument provides a wider range of choices for the material of the plate-shaped main body 5. As shown in FIG. 2, the resin layer 11 preferably has a shape with a gradual change in thickness, with a lowermost section 14 and an uppermost section 16 thicker than a center section 18 in the height direction of the side wall of the groove 9. If it has such a shape, the cross-section of the blood flow channel 7 becomes more like a circle or an oval, which makes the flow of blood smoother and effectively prevents clot formation. Note that the terms height direction, lowermost section and uppermost section are for convenience in describing the resin layer, and it is not necessary to use the medical instrument with the lowermost section downward. This shape can be formed by utilizing the surface tension of the resin according to the manufacturing method of the medical instrument described later. The width of the resin layer 11 (width of the area of the center section 18) may be adjusted as appropriate according to the width of the groove 9 (flow channel), the type of the resin, etc. For example, the width of the resin layer 11 is 0.01 mm or more and 4 mm or less, preferably 0.01 mm or more and 2 mm or less. The lowermost section 14 and the uppermost section 16 of the resin layer are preferably thicker than the center section 18 of the resin layer to make the shape of the resin layer smooth, and the lowermost section 14 and the uppermost section 16 of the resin layer is preferably 0.01 mm or more and 4 mm or less (more preferably, 0.05 mm or more and 1 mm or less) thicker than the center section 18 of the resin layer. The lowermost section 14 and the uppermost section 16 of the resin layer are connected to the lowermost section 14 and the uppermost section 16 of an adjacent resin layer, respectively, and the resin layer may cover the periphery of the groove 9.

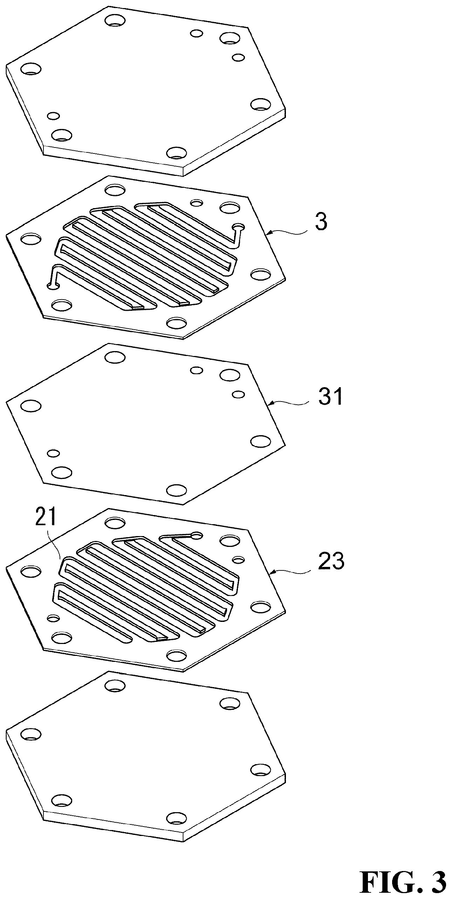

FIG. 3 is a conceptual diagram for describing the layer structure of the blood filtration device and a blood separation device. As shown in FIG. 3, the medical instrument further includes the filtrate flow channel layer 23 and the filtration film 31.

### Filtrate flow channel layer 23

The filtrate flow channel layer 23 refers to the layer including the filtrate flow channel 21 that is arranged along the blood flow channel 7.

### Filtration film 31

The filtration film 31 is interposed between the blood flow channel layer 3 and the filtrate flow channel layer 23 to filter blood that is flowed through the blood flow channel 7. The filtration film filters blood to remove harmful substances and excessive water, composed of a polymer film including many nanoscale pores, and in this example, a PES (polyethersulfone) film is used. The PES film can selectively filter only blood components with small molecular weight utilizing nanoscale pores, thereby removing harmful substances and excessive water from blood.

### Manufacturing method of the medical instrument

The method includes a resin applying step (S101), a bonding step (S103) and a post-curing step (S104). A pre-curing step (S102) may be included between the resin applying step (S101) and the bonding step (S103), or between the bonding step (S103) and the post-curing step (S104). FIG. 4 is a conceptual diagram for describing manufacturing processes of the medical instrument.

### Resin applying step (S101)

The resin applying step (S101) is a step of applying the resin to the groove 9 of the plate-shaped main body 5 that includes the groove 9 for forming the blood flow channel 7. Examples of resins include a thermosetting resin and a photocurable resin. FIG. 4 (a) is a diagram illustrating an example of the cross-section of the plate-shaped main body including the groove. The plate-shaped main body 5 including the groove 9 shown in FIG. 4 (a) can be prepared as appropriate using, for example, the methods described in Japanese Patent Publication Nos. 2017-158814 and 2020-22524. The method of applying the resin 10 to the groove 9 is publicly known. The applying methods include dip coating and spray coating. FIG. 4 (b) is a conceptual diagram illustrating the groove being applied with the resin. In this figure, the resin is bonded to a substrate main body 5. For dip coating, the plate-shaped main body may be dipped into the resin. After the plate-shaped main body is fully dipped, the plate-shaped main body may be removed from the resin to remove excessive resin from the groove. The conditions under which the plate-shaped main body is dipped into the resin (environment, orientation of the plate-shaped main body, dipping speed, etc.), dipping time, conditions under which the plate-shaped main body is removed from the resin, and the method of removing excessive resin may be adjusted as appropriate.

### Pre-curing step (S102)

The pre-curing step (S102) is an optional step of curing the resin applied to the groove 9 in the resin applying step. In this step, the resin may not be completely cured but just cured to the extent that the resin loses its fluidity. The pre-curing step may be omitted. FIG. 4 (c) is a diagram illustrating an example of the pre-curing step. If the resin is a photocurable resin, as shown in FIG. 4 (c), the resin can be cured by irradiating light (hv) onto the resin. The light irradiation time may be adjusted as appropriate according to the types of the photocurable resin and the light irradiation device that are used. For example, the light irradiation time may be 1 s or longer and 20 s or shorter, or 2 s or longer and 15 s or shorter. When the resin is a thermosetting resin, the resin can be cured by heating the resin. The pre-curing step (S102) may be performed without fully curing the resin. When the resin is a photocurable resin and light of the same intensity is used in the pre-curing step and the post-curing step, for example, light may be irradiated in the pre-curing step of a period of 1/100 or longer and 1/5 or shorter of the period in the post-curing step. Before sandwiching the main body 5 between films 41 and 42, the resin may be slightly cured in advance in the pre-curing step, and after sandwiching the main body 5 between the films 41 and 42, the resin applied to the films 41 and 42 may be cured in the post-curing step.

### Bonding step (S103)

The bonding step (S103) is a step of bonding the plate-shaped main body 5, a first film 41 and a second film 43 by sandwiching the plate-shaped main body 5 between the first film 41 and the second film 43. FIG. 4 (d) is a conceptual diagram illustrating the plate-shaped main body being sandwiched between the first film and the second film. For example, the first film 41 and the second film 43 include a filtration film 31 and a dialysis film. The films 41 and 43 preferably contain water and are not dry. If the films 41 and 43 are dry, the resin may enter the pores inside the films due to capillary action, and the pores may clog. Thus, including a step of adding water to the films 41 and 43 is a preferable example of the bonding step. To add water to the films 41 and 43, the films 41 and 43 may be dipped in water, or water may be applied to the films 41 and 43 by spraying, etc. Since the films 41 and 43 are flexible, for example, the films 41 and 43 may be fixed to a substrate and then bonded to the plate-shaped main body. For example, a polyethersulfone (PES) film is an asymmetric film including nanopores (dense layer) and micropores (support layer). In such a case of a film with a dense layer and a support layer, the dense layer is preferably on the side of the plate-shaped main body 5.

The plate-shaped main body 5 is preferably sandwiched between the first film 41 and the second film 43, and then held at rest. When this process is performed, pressing the plate-shaped main body 5 sandwiched between the first film 41 and the second film 43 may cause the resin present in the plate-shaped main body 5 to penetrate into the flow channels and the pores of the films.

### Post-curing step (S104)

The post-curing step (S104) is a step of curing the resin that is applied to the groove 9 in the resin applying step after the bonding step. If the resin is a photocurable resin, the resin can be cured by irradiating light onto the resin. FIG. 4 (e) is a conceptual diagram illustrating how the resin applied to the groove is cured by irradiating light (hv) onto the plate-shaped main body obtained in the bonding step that is sandwiched between the first film and the second film. When the resin is a thermosetting resin, the resin can be cured by heating the resin. The light irradiation time may be adjusted as appropriate according to the types of the photocurable resin and the light irradiation device that are used. For example, the light irradiation time may be 30 s or longer and 10 min or shorter, or 1 min or longer and 5 min or shorter. FIG. 4 (f) is a conceptual diagram illustrating a partial view of the medical instrument obtained.

When the medical instrument includes the blood flow channel layer and the filtrate flow channel layer, it is preferable to form the filtrate flow channel layer after the blood flow channel layer is formed. Meanwhile, the blood flow channel layer may be formed after the filtrate flow channel layer is formed beforehand. For the blood flow channel layer, the presence or absence of bubbles in an adhesive has a significant impact on the surface condition of the flow channel, thus it is preferable to check for the presence or absence of bubbles by optical transmission or ultrasonic waves after confirming a set. If the filtrate flow channel layer is formed prior to the blood flow channel layer, this check is harder to perform. From this perspective, when the medical instrument includes the blood flow channel layer and the filtrate flow channel layer, it is preferable to manufacture the blood flow channel layer prior to the filtrate flow channel layer. Since the filtrate flow channel of the filtrate flow channel layer is not a flow channel through which blood is flowed, the resin layer may not be formed in the flow channel. The blood flow channel layer and the filtrate flow channel layer may, for example, be bonded using an adhesive sheet.

### EXAMPLE

The medical instrument was manufactured in the following manner. All steps were performed in a positive-pressure clean room or a clean bench of Class 100,000 or better. They may be performed by aseptic manipulation, or final sterilization may be performed. Especially in the case of mass manufacturing of the medical instruments, the order of each step may significantly vary.

### Step 1

A blood flow channel base and a filtrate flow channel base were cut out from a 300 µm PMMA plate (cast) using a laser cutting machine. The distance between a laser and the PMMA plate was 5 mm using laser settings with a power of 20.00 W, speed of 0.3 inch/s and PPI of 30,000 Hz.

### Step 2

An adhesive pool was prepared by adding sufficient drops of a UV-curable resin (Henkel UV-curable adhesive LOCTITE 4304 (main ingredient: photocurable ethyl cyanoacrylate)) to a glass petri dish.

### Step 3

Water was removed from the surface of a dialysis film (made of polyethersulfone, 100 x 10 x 0.1 mm) to be stored in water, and the surface tension of the water on a glass plate (100 x 100 mm) was used to fix the dialysis film to the glass plate. Two sets of these were prepared. When this process was performed, if the dialysis film was completely dried, the strength of the film in the final product would deteriorate, thus only excessive water on the surface was removed.

If the dialysis film is dry, adhesive liquid may be carried through the pores inside the film due to capillary action and may completely clog the pores, thus the process was performed with great care.
Since a polyethersulfone (PES) film is an asymmetric film including nanopores (dense layer) and micropores (support layer), the nanopores were placed in the front on the glass plate.

### Step 4

The blood flow channel base was dipped into an adhesive pool with a pair of tweezers.

### Step 5

After the blood flow channel base was completely dipped into the adhesive pool, the blood flow channel base was removed, and an excessive adhesive was removed using gravity (and a spatula, etc., if necessary).

### Step 6

The adhesive was pre-cured with a UV exposure machine (14 +/- 4 W/m²). The pre-curing time was set to 10 s or shorter. Note that the manufacturing was, in practice, possible even without the pre-curing step.

### Step 7

The blood flow channel base after the pre-curing step was bonded with the PES film prepared in step 3 together to the glass plate. When this process was performed, only gravity was used for the bonding without pressing. It is expected that pressing would cause the adhesive to greatly penetrate into the flow channel, narrowing the width of the flow channel.

### Step 8

The blood flow channel base that was bonded and the PES film were irradiated with the UV exposure machine (14 +/- 4 W/m²) for 120 s or longer for post-curing.

### Step 9

A blood flow channel set (PES film - blood flow channel layer - PES film) was stored in water to prevent damage due to drying.

### Step 10

The final stacking structure was an alternating arrangement of "PES film - blood flow channel layer - PES film - filtrate flow channel layer - PES film - blood flow channel layer..." The filtrate flow channel base was dipped into the adhesive pool in the same manner as in step 4, and the filtrate flow channel base was removed to remove the excessive adhesive. Note that since the filtrate flow channel does not contact with blood, the manufacturing method of the flow channel may be performed without using a UV-curable adhesive.

### Step 11

The removed filtrate flow channel base was bonded to the blood flow channel set and cured by irradiating with the UV exposure machine (14 +/- 4 W/m²) for 240 s or longer.

### Step 12

The filtrate flow channel base after pool dipping was bonded with the stacking structure of "PES film - blood flow channel layer - PES film - filtrate flow channel layer - PES film - blood flow channel layer - PES film" and the blood flow channel set manufactured in step 11.

### Step 13

A stacked flow channel was formed by repeating steps 10 through 12.

### Step 14

A support section of each flow channel base was removed.

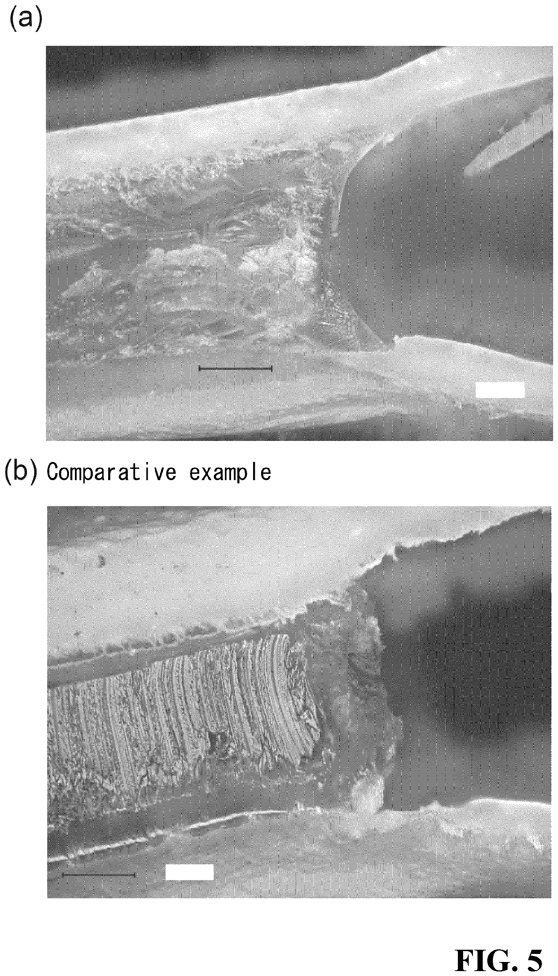

A groove section of the medical instrument obtained as described above was photographed. The obtained image is shown in FIG. 5. FIG. 5 (a) is a photograph in lieu of a drawing of the groove section obtained in the example. FIG. 5 (b) is a photograph in lieu of a drawing of the groove section where the resin layer is not provided. As shown in FIG. 5 (b), without the resin layer, the groove section is rectangular, resulting in clot formation in this section. In contrast, as shown in FIG. 5 (a), with the resin layer, the corners become rounded. As a result, it is considered that the resin layer reduces the possibility of clot formation.

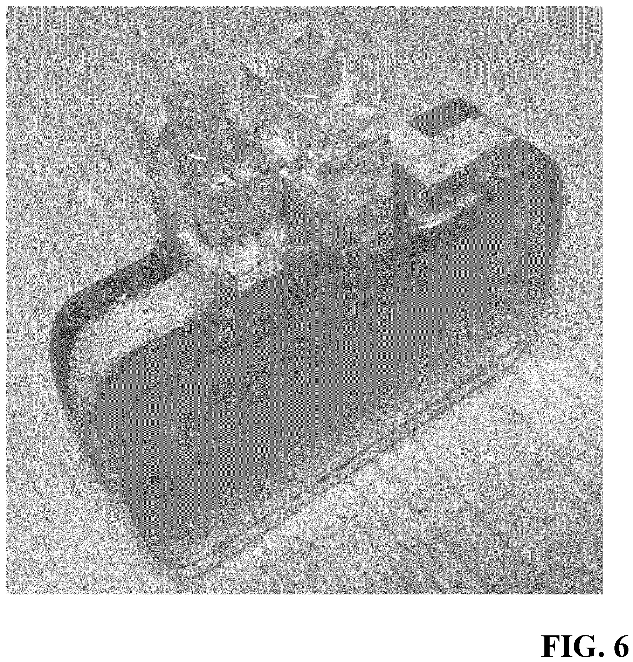

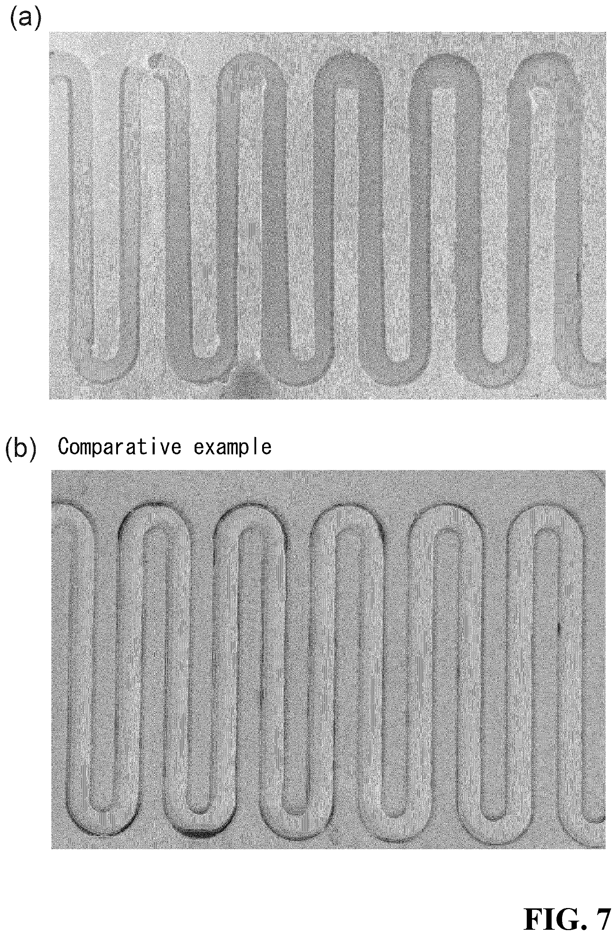

FIG. 6 is a photograph in lieu of a drawing showing the appearance of the medical instrument obtained. Blood was filtered using this medical instrument (blood filtration device). The result is shown in FIG. 7. FIG. 7 (a) is a photograph in lieu of a drawing showing how the blood flow channel layer is removed after using the medical instrument obtained in the example. FIG. 7 (b) is a photograph in lieu of a drawing showing how the medical instrument that does not include the resin layer is used. As shown in FIG. 7 (b), without the resin layer, clots were formed. In contrast, as shown in FIG. 7 (a), with the resin layer, clots were not formed in the blood flow channel layer.

### INDUSTRIAL APPLICABILITY

The present invention may be used in the field of medical instruments.

### REFERENCE SIGNS LIST

- 1: Medical instrument
- 3: Blood flow channel layer
- 5: Plate-shaped main body
- 7: Blood flow channel
- 9: Groove
- 11: Resin layer
- 21: Filtrate flow channel
- 23: Filtrate flow channel layer
- 31: Filtration film
- 41: First film
- 43: Second film

## Claims

1. A medical instrument (1) comprising
a plurality of blood flow channel layers (3),
wherein each of the plurality of blood flow channel layers (3) includes:
a plate-shaped main body (5);
a groove (9) formed in the plate-shaped main body (5) for forming a blood flow channel (7); and
a resin layer (11) formed on a side wall of the groove (9),
wherein
the resin layer (11) has a shape with a gradual change in thickness, and the shape has a lowermost section and an uppermost section thicker than a center section in a height direction of the side wall of the groove (9).

2. The medical instrument (1) according to claim 1, further comprising:
a filtrate flow channel layer (23) including a filtrate flow channel (21) arranged along the blood flow channel (7); and
a filtration film (31) interposed between the blood flow channel layer (3) and the filtrate flow channel layer (23) to filter blood flowing through the blood flow channel (7).

3. The medical instrument (1) according to claim 1, wherein the medical instrument is an implantable artificial kidney, a portable artificial kidney, a hemodialysis machine, a blood filtration device, or an extracorporeal membrane oxygenator.

4. A manufacturing method of a medical instrument, comprising:
a resin applying step of applying a resin to a groove (9) of a plate-shaped main body (5) that includes the groove (9) for forming a blood flow channel (7);
a bonding step of bonding the plate-shaped main body (5), a first film (41), and a second film (43) by sandwiching the plate-shaped main body (5) between the first film (41) and the second film (43); and
a post-curing step of curing the resin that is applied to the groove (9) in the resin applying step after the bonding step.

5. The manufacturing method of a medical instrument according to claim 4,
wherein the medical instrument is the medical instrument according to any one of claims 1 to 3.

6. The manufacturing method of a medical instrument according to claim 5,
wherein the resin is a photocurable resin, and the post-curing step is a step of curing the resin by irradiating light onto the resin.

7. The manufacturing method of a medical instrument according to claim 6, further comprising:
a pre-curing step of curing the resin that is applied to the groove (9) in the resin applying step by irradiating light onto the resin,
wherein the bonding step is a step after the pre-curing step, and
the pre-curing step is a step of irradiating the light for a period of 1/100 or longer and 1/5 or shorter of the period in the post-curing step.
